# EUROPEAN PATENT SPECIFICATION

(11) **EP 1 380 283 B1**
(45) Date of publication and mention of the grant of the patent: **23.03.2011**
(21) Application number: 03254406.6
(22) Date of filing: 11.07.2003
(51) Int. Cl.: A61K 8/34, A61K 8/46, A61K 8/42, A61K 8/45, A61K 8/86, A61Q 19/00, A61K 8/894

(54) **Cosmetic composition**
Kosmetische Zusammensetzung
Composition cosmétique

(30) Priority: 11.07.2002 JP 2002202145
(43) Date of publication of application: 14.01.2004
(73) Proprietor: Ajinomoto Co., Inc., Tokyo 104-8315 (JP)
(72) Inventor: Nakanishi, Noriyuki Ajinomoto Co., Inc., Kawasaki-shi Kanagawa 210-8681 (JP)
(74) Representative: Nicholls, Kathryn Margaret

(56) References cited:
- EP-A- 0 267 707
- GB-A- 2 217 342
- US-A1- 2002 028 187
- US-B1- 6 190 645
- US-B1- 6 417 145
- RESEARCH DISCLOSURE, no. 451030, 1 November 2001 (2001-11-01), Westbourne, UK
- SCIENTIFIC LITERATURE REVIEW, COSMETIC INGREDIENT REVIEW, 30 April 2003 (2003-04-30), Washington, US

## Description

The present invention relates to the use of t-butanol in a cosmetic composition. More specifically, the present invention relates to the use of t-butanol in a cosmetic composition which comprises a surfactant having an oxyethylene group in the molecule.

Surfactants having an oxyethylene group (-CH₂CH₂O-) in the molecule are widely used in cosmetics such as skin washing agents, hair washing agents, and emulsifying agents. For example, alkylether sulfates are used for skin washing agents or hair washing agents, end polyoxyethyleneglyceryl esters and polyoxyethylenealkyl ethers and the like are commonly used as emulsilfying agents. However, when a surfactant having an oxyethylene group in the molecule is mixed in cosmetics, a problem arises in that the presence of an oxyethylene group may become a cause of smell change or odor generation with passage of time. Masking with fragrance, formulation with a chelate agent or an antioxidant and the like have so far been employed to solve the problem of the smell change or the odor generation.

Cosmetic preparations comprising surfactants having oxyethlylene groups and denatured alcohol are known from US6190645, US2002/0028187 and 982217342.

An object of the present invention is to provide a cosmetic composition which comprises a surfactant having an oxyethylene group in the molecule, and wherein the smell change or the odor generation with passage of time are reduced or eliminated. The inventor of the present invention conducted intensive researches to achieve the foregoing object, and as a result, they found that the smell change or the odor generation with passage of time are significantly suppressed by addition of tert-butanol to a cosmetic comprising a surfactant having an oxyethylene group in the molecule. The present invention was achieved on the basis of these findings.

The present invention thus provides the use of tert-butanol in a cosmetic composition comprising one or more surfactants having an oxyethylene group in the molecule, the tert-butanol being included in the composition in an amount of 0.01-1000 ppm based on total weight of the composition, for suppressing change of smell of, or odour generation in, the composition with the passage of time.

According to preferred embodiments of the aforementioned invention, having an oxyethylene group in the molecule is included in an amount of from 1 to 20 weight % based on the total weight of the cosmetic composition.

According to further preferred embodiment of the present invention, there is provided the aforementioned use wherein the surfactant having an oxyethylene group in the molecule is one or more types of surfactants selected from the group consisting of a surfactant represented by the following general formula (1):

[R¹(OCH₂CH₂)ₙ-OSO₂]⁻M⁺

(wherein R¹ represents a linear or branched alkyl group having 7 to 21 carbon atoms or a linear or branched alkenyl group having 7 to 21 carbon atoms, n represents an integer of 1 to 30, and M represents Na, K, NH₄, or triethanolamine);
a surfactant represented by the following general formula (2):

R²CO-NH(CH₂OH₂O)ₘH

(wherein R² represents a linear or branched alkyl group having 7 to 21 carbon atoms or a linear or branched alkenyl group having 7 to 21 carbon atoms, and m represents an integer of 1 to 10);
a surfactant represented by the following general formula (3):

R³CO-N (CH₂CH₂OH)₂

(wherein R² represents a linear or branched alkyl group having 7 to 21 carbon atoms or a linear or branched alkenyl group having 7 to 21 carbon atoms).

When a cosmetic composition comprises (A) one or more types of surfactant selected from the group consisting of surfactants which have an oxyethylene group in the molecule; and (B) tert-butanol, the smell change or the odor generation with passage of time in said composition is suppressed by tert-butanol. The term "smell change or odor generation" should be construed in its broadest sense including alteration of smell in a composition; addition of bad smell; generation of some odor from an odorless composition, and should not be construed in any limitative sense. Suppression of the smell change or the odor generation includes reduction of the smell, change or the odor generation, as well as substantially complete elimination of the smell change or the odor generation.

Type of the surfactant having an oxyethylene group is not limited as far as the surfactant has one or more oxyethylene groups in the molecule. The oxyethylene group contained in the molecule is preferred to be a polyoxyethylene group. More specifically, examples include a polyoxyethylenealkyl ether sulfate, a polyoxyethylenenonylphenyl ether sulfate, a polyoxyethylenealkylsulfo succinate, a polyoxyethylenealkyl ether acetate, a polyoxyethylenealkyl ether phosphoric acid, a polyoxyethylenealkylphenyl ether phosphoric acid, a fatty acid diethanolamide, a fatty acid monoethanolamide, a polyethylene glycol distearate, a polyoxyethylenealkyl ether, a polyoxyethylenenonylphenyl ether, a fatty acid polyoxyethylenealkyl ether, a fatty acid polyoxyethyleneglycerol ether, a polyoxyethylene hardened castor oil, a fatty acid polyoxyethylene hardened castor oil, a fatty acid polyoxyethylene sorbitan, or a polyoxyethylene-methylpolysiloxane copolymer. A single surfactant having an oxyethylene group may be used, or a combination of two or more surfactants may also be used.

Most preferred classes of the surfactant having an oxyethylene group are one or more types of surfactants selected from the group consisting of an alkyl ether sulfate represented by the following general formula (1):

[R¹(OCH₂CH₂)ₙ-OSO₈]⁻M⁺

(wherein R¹ represents a linear or branched alkyl group having 7 to 21 carbon atoms or a linear or branched alkenyl group having 7 to 21 carbon atoms, n represents an integer of 1 to 30, and M represents Na, K, NH₄, or triethanolamine);
an alkanolamide represented by the following general formula (2):

R²CO-NH(CH₂CH₂O)ₘH

(wherein R² represents a linear or branched alkyl group having 7 to 21 carbon atoms or a linear or branched alkenyl group having 7 to 21 carbon atoms, and m represents an integer of 1 to 10);
an N-acyl diethanolamide represented by the following general formula (3):

R⁸CO-N (CH₂CH₂OH)₂

(wherein R⁸ represents a linear or branched alkyl group having 7 to 21 carbon atoms or a linear or branched alkenyl group having 7 to 21 carbon atoms).

In the above general formulae, the alkyl group or the alkenyl group having 7 to 21 carbon atoms represented by R¹, R², and R⁸ may be linear or branched. When a branched alkyl group is used, the number of branch is not particularly limited. The number of double bonds in the alkenyl group is not particularly limited, and the number is generally about 1 to 4, and preferably about 1 to 2. When the alkenyl group has two or more double bonds, they may be conjugated double bonds.

In the general formula (1), R¹ is preferred to be a linear or branched alkyl group having 11 to 15 carbon atoms or a linear or branched alkenyl group having 11 to 15 carbon atoms, and M is preferred to be Na, NK₄, or triethanolamine. More specifically, for example, sodium polyoxyethylene (8) alkyl (12-14) ether sulfate, sodium polyoxyethylene (2) alkyl (12, 13) ether sulfate or the like is preferred,

In the general formula (2), R² is preferred to be a linear or branched alkyl group having 9 to 18 carbon atoms or a linear or branched alkenyl group having 9 to 18 carbon atoms. More preferably R² is a linear or branched alkyl group having 10 to 14 carbon atoms or a linear or branched alkenyl group having 10 to 14 carbon atoms and m is 1. More specifically, for example, castor oil fatty acid monoethanol amide or lauric acid monoethanol amide or the like is preferred.

In the general formula (8), R⁸ is preferred to be a linear or branched alkyl group having 9 to 18 carbon atoms or a linear or branched alkenyl group having 9 to 18 carbon atoms. More specifically, for example, castor oil fatty acid diethanol amide or lauric acid diethanol amide or the like ie preferred.

A content of the surfactant having an oxyethylene group in the molecule in the cosmetic composition can be suitably determined by those ordinary skilled in the art depending on type of the cosmetic, desired properties and the like, and the content is not particularly limited. Generally, the content may be in the range of 0.1 to 50 weight %, preferably 1 to 20 weight % based on the total weight of the cosmetic composition. When the content of the aforementioned surfactant is less than 1 weight %, the problem of the smell change or the odor generation with passage of time is generally reduced, and thus the addition of tert-butanol may not be needed for solving the problem.

A content of tert-butanol in the cosmetic composition is from 0.01 to 1,000 weight ppm based on the total weight of the composition. When the content of tert-butanol is less than 0.01 weight ppm, suppression of the smell change or the odor generation with passage of time may sometimes be insufficient. When the content is more than 1,000 weight ppm, odor inherent to tert-butanol may sometimes cause a problem.

The type of the cosmetic composition is not particularly limited. For example, the composition may be used as hair washing agents such as a shampoo, a hair rinse, a 2-in-1 shampoo, and a conditioning shampoo, various hair cosmetics such as a hair lotion, a hair conditioner, a hair treatment, hair cream, a hair lacquer, a hair liquid, a hair wax, a hair water, a hair styling agent, a perm solution, a hair coloring, an acidic hair coloring, a hair manicure, or various skin cosmetics such as a skin lotion, a milky lotion, a facial washing agent, a makeup remover, a cleansing lotion, an emollient lotion, nourishing cream, emollient cream, massage cream, cleansing cream, a body shampoo, hand soap, solid soap, preshave cream, a tanning cosmetic, deodorant powder, a deodorant lotion, a deodorant spray, a makeup remover gel, a moisture gel, a moisturizing essence, an ultraviolet rays protective essence, shaving foam, face powder, a foundation, lip rouge, cheek rouge, eyeliner, eye shadow, an eyebrow pencil, and a bath preparation, or toothpaste.

For the preparation of the cosmetic composition one or more additives which are generally used for the preparation of cosmetic compositions may be used. The aforementioned additive can be used in a suitable amount in a range where the additive does not destroy the advantageous effect of the present invention. For example, additives such as surfactants such as an anionic surfactant, a cationic surfactant, an amphoteric surfactant, and a nonionic surfactant, waxes, a vegetable oil, animal oil and fat, a derivative of natural oil and fat, mineral oil and fat, a lower and higher fatty acid ester, a synthetic oil and fat such as N-acyl glutamic acid ester, a polymer material, an alcohol, a polyhydric alcohol, an extract, an amino acid, a nucleic acid, a vitamin, a hydrolyzed protein and their derivatives, a glycerylorate, an enzyme, an anti-inflammatory agent, a disinfectant, an antiseptic, an anti-oxidant, an absorbent of ultraviolet rays, a chelating agent, an antihidrotic, an oxidative dye, a pH modifier, a pearling agent, and a wetting agent can be used. A method for preparation of the cosmetic composition of the present invention is not particularly limited. The aforementioned ingredient (A) and ingredient (B) may be mixed by a suitable means, added and mixed optionally with one or more of the aforementioned additives, and thus, the cosmetic composition can be easily prepared by the method well known to those skilled in the art. The order of addition of each ingredient and the procedure of the obtained composition are not particularly limited.

### Examples

Embodiments of the present invention will be explained more specifically, by way of illustration only, by referring to the following examples.

### Test example 1

A shampoo of the formulation shown in Table (shown in weight %, total amount 100 %) was prepared by an ordinary method, and the odor after a 6-month storage at 40°C was evaluated. The evaluation was conducted based on the following criteria to calculate average values. The average value of 2.5 or more was represented as inferior (×), 1.6 to 2.4 was represented as slightly inferior (Δ), 1.4 or less was represented as superior (○). The results of the evaluation are shown in Table 1, Comparative Examples 2 and 3 were prepared as compositions which was free from a surfactant having polyoxyethylene group in the molecule.

### [Criteria for evaluation]

### Odor after storage

3: Significant smell change or odor generation compared to the smell before the storage
2: Little smell change or odor generation compared to the smell before the storage
1: No smell change or odor generation compared to the smell before the storage

**Table 1**

| Ingredient | Example 1 | Example 2 | Comparative Example 1 | Comparative Example 2 | Comparative Example 3 |
|---|---|---|---|---|---|
| Sodium polyoxyethylene (2) lauryl ether sulfate | 9.0 | 9.0 | 9.0 | | |
| Sodium lauryl sulfate | | | | 9.0 | 9.0 |
| Cocamidepropylbetaine | 4.5 | 4.5 | 4.5 | 4.5 | 4.5 |
| Sodium chloride | 1.0 | 10 | 1.0 | 1.0 | 1.0 |
| Sodium PCA | 1.0 | 1.0 | 1.0 | 1.0 | 1.0 |
| Butylene glycol | 3.0 | 3.0 | 3.0 | 3.0 | 3.0 |
| Polyquaternium-10 | 0.2 | 0.2 | 0.2 | 0.2 | 0.2 |
| Methylparaben | 0.2 | 0.2 | 02 | 02 | 0.2 |
| Tert-Butanol (0.1% aqueous solution) | 0.50 | 0.05 | | 0.50 | |
| Water | the rest | the rest | the rest | the rest | the rest |
| total | 100.0 | 100.0 | 100.0 | 100.0 | 100.0 |
| Odor evaluation after storage | ○ | ○ | × | ○ | ○ |

### Test example 2

A body shampoo of the formulation shown in Table 2 (shown in weight %, total amount 100 %) was prepared by an ordinary method, and the odor after a 6-month storage at 40°C was evaluated. The evaluation was conducted based on the following criteria to calculate average values. The average value of 2.5 or more was represented as inferior (×), 1.5 to 2.4 was represented as slightly inferior (Δ), 1.4 or less was represented as superior (O). The results of the evaluation are shown in Table 2.

### [Criteria for evaluation]

### Odor after storage

8: Significant smell change or odor generation compared to the smell before the storage
2: Little smell change or odor generation compared to the smell before the storage
1: No smell change or odor generation compared to the smell before the storage

**Table 2**

| Ingredient | Example 3 | Example 4 | Comparative Example 4 |
|---|---|---|---|
| Lauric acid | 6.0 | 8.0 | 6.0 |
| Myristic acid | 4.0 | 4.0 | 4.0 |
| Palmitic said | 2.0 | 2.0 | 2.0 |
| Potassium hydroxide | 3.4 | 3.4 | 3.4 |
| Propylene glycol | 6.0 | 6.0 | 6.0 |
| Leuramide DEA | 3.0 | 3.0 | 3.0 |
| Cocamidepropylbetaine | 3.0 | 3.0 | 3.0 |
| Hydroxypropylmethylcellulose | 0.2 | 0.2 | 0.2 |
| Glycol distearate | 2.0 | 2.0 | 2.0 |
| Tert-Butanol (0.1% aqueous solution) | 0.05 | 0.005 | |
| Water | the rest | the rest | the rest |
| total | 100.0 | 100.0 | 100.0 |
| Odor evaluation after storage | ○ | ○ | × |

### Test example 8

A milky lotion of the formulation shown in Table 3 (shown in weight %, total amount 100 %) was prepared, and the odor after a 6-month storage at 40 °C was evaluated. The evaluation was conducted based on the following criteria to calculate average values. The average value of 2.5 or more was represented as inferior (×), 1.5 to 2.4 was represented as slightly inferior (Δ), 1.4 or less was represented as superior (O). The results of the evaluation are shown in Table S.

### [Criteria for evaluation]

### Odor after storage

3: Significant smell change or odor generation compared to the smell before the storage
2: Little smell change or odor generation compared to the smell before the storage
1: No smell change or odor generation compared to the smell before the storage

**Table 3**

| Ingredient | Example 5 | Comparative Example 5 |
|---|---|---|
| Liquid paraffin | 5.0 | 5.0 |
| Dioctyldodecyl lauroyl glutamate | 2.0 | 2.0 |
| Propylene glycol stearate | 0.5 | 0.5 |
| PEG-5 hydrogenated oastor oil | 1.5 | 1.5 |
| PEG-5 glyceryl stearate | 2.5 | 2.5 |
| Carbomer | 0.2 | 0.2 |
| Sodium hydroxide | 0.08 | 0.08 |
| Butylene glycol | 5.0 | 5.0 |
| Methylparaben | 0.1 | 0.1 |
| Tert-Butanol (0.1% aqueous solution) | 0.02 | |
| Water | the rest | the rest |
| total | 100.0 | 100.0 |
| Odor evaluation after storage | ○ | Δ |

### Test example 4

A milky lotion of the formulation shown in Table 4 (shown in weight %, total amount 100 %) was prepared, and the odor after a 6-month storage at 40 °C was evaluated. The evaluation was conducted based on the following criteria to calculate average values. The average value of 2.5 or more was represented as inferior (×), 1.5 to 2.4 was represented as slightly inferior (Δ), 1.4 or less was represented as superior (O), The results of the evaluation are shown in Table 4.

### [Criteria for evaluating]

### Odor after storage

8: Significant smell change or odor generation compared to the smell before the storage
2: Little smell change or odor generation compared to the smell before the storage
1: No smell change or odor generation compared to the smell before the storage

**Table 4**

| Ingredient | Example 6 | Example 7 | Comparative Example 6 | Comparative Example 7 | Comparative Example 8 |
|---|---|---|---|---|---|
| Cyclomethicone | 20.0 | 20.0 | 20.0 | 20.0 | 20.0 |
| Polyoxyethylene methylpolysiloxane copolymer | 3.0 | 3.0 | 3.0 | | |
| Glyceryl diisostearate | | | | 3.0 | 3.0 |
| Micro Particle Titanium Dioxide | 10.0 | 10.0 | 10.0 | 10.0 | 10.0 |
| Sodium PCA | 1.0 | 1.0 | 1.0 | 1.0 | 1.0 |
| Butylene glycol | 10.0 | 10.0 | 10.0 | 10.0 | 10.0 |
| Phenoxyethanol | 0.2 | 0.2 | 0.2 | 0.2 | 0.2 |
| Tert-Butanol (0.1% aqueous solution) | 1.0 | 0.1 | | 0.1 | |
| Water | the rest | the rest | the rest | the rest | the rest |
| total | 100.0 | 100.0 | 100.0 | 100.0 | 100.0 |
| Odor evaluation after storage | ○ | ○ | × | ○ | ○ |

Example 8: Preparation of a shower gel

A shower gel was prepared according to the following formulation by an ordinary method. The obtained shower gel was free from smell change or odor generation with passage of time, and highly stable.

**Table 5**

| Ingredient | Example 8 |
|---|---|
| Ammonium polyoxyethylene (3) lauryl ether sulfate | 9.0 |
| Cocamidepropylbetaine | 4.5 |
| Disodium cocoyl glutamate | 0,5 |
| Leureth-2 | 1.5 |
| Sodium PCA | 1.0 |
| Polyquaternium-10 | 0.4 |
| Methylparaben | _ 0.2 |
| tert-Butanol (0.1% aqueous solution) | 1.0 |
| Fragrance | suitable amount |
| Water | the rest |
| total | 100.0 |

### Example 9: Preparation of a hair conditioner

A hair conditioner was prepared according to the following formulation by an ordinary method. The obtained hair conditioner was free from smell change or odor generation with passage of time, and highly stable.

**Table 6**

| Ingredient | Example 9 |
|---|---|
| Steartrimonium chloride | 1.00 |
| Cetyl alcohol | 4.00 |
| Hexyldecyl isostearate | 2.00 |
| Dimethicone | 2.00 |
| Steareth-40 | 2.00 |
| Lactic acid | 0.01 |
| Sodium PICA | 1.00 |
| Disodium EDTA | 0.06 |
| Methylparaben | 0.20 |
| Tert-Butanol-(0.1% equeous solution) | 1.00 |
| Fragrance | suitable amount |
| Water | the rest |
| total | 100.00 |

### Example 10: Preparation of facial cleansing foam

Facial cleansing foam was prepared according to the following formulation by an ordinary method. The obtained facial cleansing foam was free from smell change or odor generation with passage of time, and highly stable.

**Table 7**

| Ingredient | Example 10 |
|---|---|
| Lauric acid | 8.00 |
| Myristic acid | 8.00 |
| Palimitic | 8.00 |
| Stearic acid | 10.00 |
| Glycerol | 25.00 |
| Butylene glycol | 8.00 |
| Cocamide MEA | 2.00 |
| Glyceryl stearate | 1.00 |
| Potassium hydroxide | 6.95 |
| Cocamidepropylbetaine | 1.00 |
| Potassium cocoyl glycinate | 3.00 |
| Methylparaben | 0.20 |
| Tert-Butanol (0.1X aqueous solution) | 1.00 |
| Fragrance | suitable amount |
| Water | the rest |
| total | 100.0 |

### Industrial Applicability

Use of tert-butanol in a cosmetic composition in accordance with the present invention, suppresses the smell change or odor generation with passage of time, which is caused by addition of a surfactant having oxyethylene group in the molecule. Therefore, the composition has high storage stability.

## Claims

1. use of tert-butanol in a cosmetic composition comprising one or more surfactants having an oxyethylene group in the molecule, the tert-butanol being included in the composition in an amount of 0.01 - 1000 ppm based on total weight of the composition, for suppressing change of smell of, or odour generation in, the composition with the passage of time.

2. The use according to claim 1, wherein the composition contains said one or more surfactants having an oxyethylene group in an amount of from 1-20% by weight based on total weight of the composition.

3. The use according to claim 1 or 2, wherein the surfactant having an oxyethylene group in the molecule is one or more types of surfactants selected from the group consisting of a surfactant represented by the following general formula (1):
[R¹(OCH₂CH₂)ₙ-OSO₃]⁻M⁺
wherein R¹ represents a linear or branched alkyl group having 7 to 21 carbon atoms or a linear or branched alkenyl group having 7 to 21 carbon atoms, n represents an integer of 1 to 30, and M represents Na, K, NH₄, or triethanolamine;
a surfactant represented by the following general formula (2):
R²CO-NH(CH₂CH₂O)ₘH
wherein R² represents a linear or branched alkyl group having 7 to 21 carbon atoms or a linear or branched alkenyl group having 7 to 21 carbon atoms, and m represents an integer of 1 to 10; and
a surfactant represented by the following general formula (3) :
R³CO-N(CH₂CH₂OH)₂
wherein R³ represents a linear or branched alkyl group having 7 to 21 carbon atoms or a linear or branched alkenyl group having 7 to 21 carbon atoms.

## Patentansprüche

1. Verwendung von tert-Butanol in einer kosmetischen Zusammensetzung, die ein oder mehrere Tenside mit einer Oxyethylengruppe im Molekül umfasst, wobei das tert-Butanol in der Zusammensetzung in einer Menge von 0,01 bis 1.000 ppm bezogen auf das Gesamtgewicht der Zusammensetzung vorhanden ist, um eine Veränderung des Geruchs der Zusammensetzung oder die Entstehung unangenehmer Gerüche in der Zusammensetzung im Lauf der Zeit zu verhindern.

2. Verwendung nach Anspruch 1, worin die Zusammensetzung das eine oder die mehreren Tenside mit einer Oxyethylengruppe in einer Menge von 1 bis 20 Gew.-% bezogen auf das Gesamtgewicht der Zusammensetzung enthält.

3. Verwendung nach Anspruch 1 oder 2, worin das Tensid mit einer Oxyethylengruppe im Molekül einem oder mehreren Tensidtypen entspricht, die aus der aus folgenden bestehenden Gruppe ausgewählt sind:
einem Tensid der folgenden allgemeinen Formel (1):
[R¹(OCH₂CH₂)ₙ-OSO₃]⁻M⁺
worin R¹ für eine unverzweigte oder verzweigte Alkylgruppe mit 7 bis 21 Kohlenstoffatomen oder eine unverzweigte oder verzweigte Alkenylgruppe mit 7 bis 21 Kohlenstoffatomen steht, n für eine ganze Zahl von 1 bis 30 steht und M für Na, K, NH₄ oder Triethanolamin steht;
einem Tensid der folgenden allgemeinen Formel (2):
R²CO-NH(CH₂CH₂O)ₘH
worin R² für eine unverzweigte oder verzweigte Alkylgruppe mit 7 bis 21 Kohlenstoffatomen oder eine unverzweigte oder verzweigte Alkenylgruppe mit 7 bis 21 Kohlenstoffatomen steht und m für eine ganze Zahl von 1 bis 10 steht; und
einem Tensid der folgenden allgemeinen Formel (3):
R³CO-N(CH₂CH₂OH)₂
worin R³ für eine unverzweigte oder verzweigte Alkylgruppe mit 7 bis 21 Kohlenstoffatomen oder eine unverzweigte oder verzweigte Alkenylgruppe mit 7 bis 21 Kohlenstoffatomen steht.

## Revendications

1. Utilisation de tert-butanol dans une composition cosmétique comprenant un ou plusieurs tensioactifs ayant un groupe d'oxyéthylène dans la molécule, le tert-butanol étant inclus dans la composition en une quantité de 0,01-1000 ppm basée sur le poids total de la composition, pour supprimer un changement olfactif de, ou une génération d'odeur dans la composition au fur et à mesure que le temps passe.

2. Utilisation selon la revendication 1, dans laquelle la composition contient lesdits un ou plusieurs tensioactifs ayant un groupe d'oxyéthylène en une quantité de 1-20% en poids basée sur le poids total de la composition.

3. Utilisation selon la revendication 1 ou 2, dans laquelle le tensioactif ayant un groupe d'oxyéthylène dans la molécule est un ou plusieurs types de tensioactifs sélectionnés dans le groupe consistant en un tensioactif représenté par la formule générale suivante (A):
[R¹(OCH₂CH₂)ₙ-OSO₃]⁻M⁺
où R¹ représente un groupe alkyle linéaire ou ramifié ayant 7 à 21 atomes de carbone ou un groupe alkényle linéaire ou ramifié ayant 7 à 21 atomes de carbone, n représente un entier de 1 à 30, et M représente Na, K, NH₄ ou triéthanolamine;
un tensioactif représenté par la formule générale suivante (2):
R²CO-NH(CH₂CH₂O)ₘH
où R² représente un groupe alkyle linéaire ou ramifié ayant 7 à 21 atomes de carbone ou un groupe alkényle linéaire ou ramifié ayant 7 à 21 atomes de carbone, et m représente un entier de 1 à 10; et
un tensioactif représenté par la formule générale suivante (3):
R³CO-N(CH₂CH₂OH)₂
où R³ représente un groupe alkyle linéaire ou ramifié ayant 7 à 21 atomes de carbone ou un groupe alkényle linéaire ou ramifié.
